(19) Europäisches Patentamt
European Patent Office
Office européen des brevets

(11) Publication number: **0 377 324 A2**

# (12) EUROPEAN PATENT APPLICATION

(21) Application number: **89313616.8**

(22) Date of filing: **27.12.89**

(51) Int. Cl.5: **C08G 73/06, C07D 401/12, C08L 23/02**

(30) Priority: **02.01.89 CS 13/89**

(43) Date of publication of application: **11.07.90 Bulletin 90/28**

(84) Designated Contracting States: **CH DE FR GB IT LI**

(71) Applicant: **SLOVENSKA AKADEMIA VIED**
**Obráncov mieru 49**
**Bratislava(CS)**

(72) Inventor: **Manasek, Zdenek**
**No. 46 Astrova**
**Bratislava(CS)**
Inventor: **Hrdlovik, Pavol**
**No. 3 Fevr. vitazstava**
**Malacky(CS)**
Inventor: **Chmela, Stefan**
**No. 50 Macov**
**Blatna na Ostrove(CS)**
Inventor: **Rychly, Jozef**
**No. 50 Astrova**
**Bratislava(CS)**
Inventor: **Danko, Pater**
**No. 3 Gercenova**
**Bratislava(CS)**
Inventor: **Karvas, Milan**
**No. 15 Gajova**
**Bratislava(CS)**
Inventor: **Vass, Frantisek**
**No. 16 Sevcenkova**
**Bratislava(CS)**
Inventor: **Durmis, Julius**
**No. 5 Ozvoldikova**
**Bratislava(CS)**
Inventor: **Masek, Jan**
**No. 28 Landauova**
**Bratislava(CS)**

(74) Representative: **Ablewhite, Alan James et al**
**MARKS & CLERK 57/60 Lincoln's Inn Fields**
**London WC2A 3LS(GB)**

## (54) Substituted polyaminotriazines and the method of their preparation.

(57) Polyaminotriazines of the formula applicable as light stabilizers

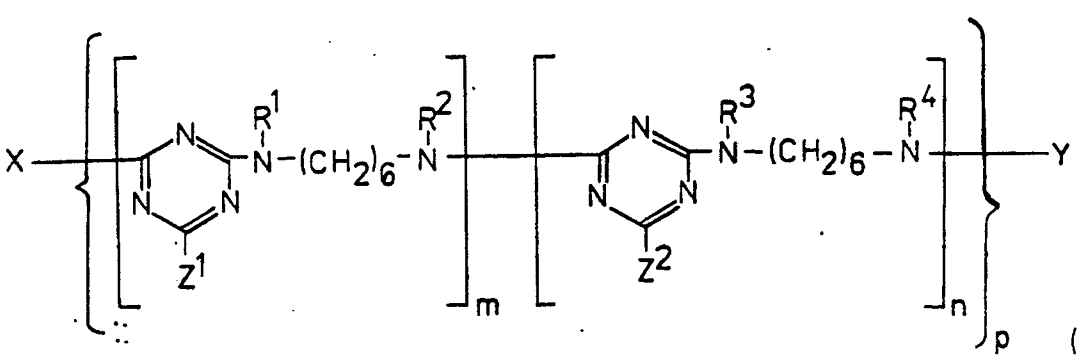

(I),

in which $R^1$ and $R^2$, either the same or different, stand for hydrogen 2,2,6,6 - tetramethyl - 4 - piperidyl group, or 1,2.2, 6,6 - pentamethyl - 4 - piperidyl group; $R^3$ and $R^4$ stand for 1 -methyl - 2 - (2,2,6,6 - tetramethyl - 4 - piperidyl) ethyl group or its 1,2,2,6,6 - pentamethyl analogue, or $R^3$ = H; $Z^1$ and $Z^2$ , either the same or different, stand for the groups; 2,2,6,6 -tetramethyl - 4 - piperidyl amino-, 1 - methyl - 2 -

EP 0 377 324 A2

(2,2,6,6 -tetramethyl - 4 - piperidyl) ethylamino-, $C_1$ - $C_{18}$ alkyl amino- , di($C_1$ - $C_4$ alkyl) amino-, cyclohexylamino-, dicyclohexyl amino, N($C_1$ - $C_4$ alkyl) cyclohexylamino-, or morpholino-. The group X = Cl, Y = H ; X and Y are also groups of the reaction component used in the excess. The molecular weight Mn varies between 1500 and 12000; m = 1-10, n = 1-8, p = 1-5. The method of preparation of polyaminotriazines of the above formula is based upon the successive condensation of 2,4 -dichloro - s - triazine (containing 5 wt.% at maximum of cyanuric chloride) occurring via the substituents $Z^1$ and $Z^2$ in position 6 with diamines $HN(R^1)(CH_2)_6NH(R^2)$ and $HN(R^3)(CH_2)_6NH(R^4)$ used either in excess or in deficit from 1 to 25 mol. %. The reaction is carried out at the temperatures from 70 to 210 °C in an inert organic solvent and in the presence of inorganic base under opto ional addition of amino compound such as $C_1$-$C_{18}$alkyl amine, cyclohexyl amine, morpholine and 2,2,6,6 - tetramethyl -4 - aminopiperidine.

## Substituted Polyaminotriazines and the Method of Their Preparation

The invention relates to substituted polyaminotriazines based on piperidyl derivatives of triazine copolymers to be used as light stabilizing agents, and to a method of their preparation.

Sterically hindered amines are considered at present to be the most efective light stabilizing agents for polymers. It is various derivatives of particularly 2,2,6,6,-tetramethylpiperidine that inhibit, with a high efficiency, degradation reactions of polymers exposed to oxygen and solar radiation. A disadvantage of light stabilizing agents on the basis of low molecular weight derivatives of sterically hindered amines resides in their volatility and extractability out of used polymers. Losses of such substances caused by their volatility are eliminated by using oligomeric stabilizing agents, and it is advantageous if the extractability thereof out of used polymers, especially polyolefins, is reduced. Polyaminotriazines belong to one of the newest groups of light stabilizers. Piperidyl derivatives of triazine polymers are light stabilizing agents that have been developed to be applied, for instance, to polypropylene fibers as well as other products made of polyolefins or other polymers. Polyaminotriazines with light stabilizing effect can be prepared either by pre-polymerization of cyanuric chloride with a corresponding N,N′-bis-(2,2,6,6-tetramethyl-4-piperidyl)alkenediamine and by the subsequent substitution of chlorine in position 6 of s-triazine, or directly by oligomerization of a derivative of piperidylalkanediamine with a derivative of 6-substituted 2,4-dichloro-1,3,5- triazine under pressure or at atmospheric pressure (BRD 2 636 144, 3 113 455, 3 117. 694, USP 4 086 204, 4 331 586, EP 24 338, 45 721, 93 693). By applying the method of atmospheric pressure, the yields are lower even in the long term reaction than those obtained in pressure process. A disadvantage of both processes is the formation of insoluble portions which are responsible for a substantial reduction of the yield. The higher content of unreacted chlorine in the oligomeric stabilizing agent may cause problems at compounding of stabilized polymeric systems at elevated temperatures as well as when using them. It is also desirable to raise the compatibility of such stabilizing agents relative used polymers.

It is an object of the present invention to eliminate the drawback of previous state of the art and to provide substituted polyaminotriazines of the general formula I

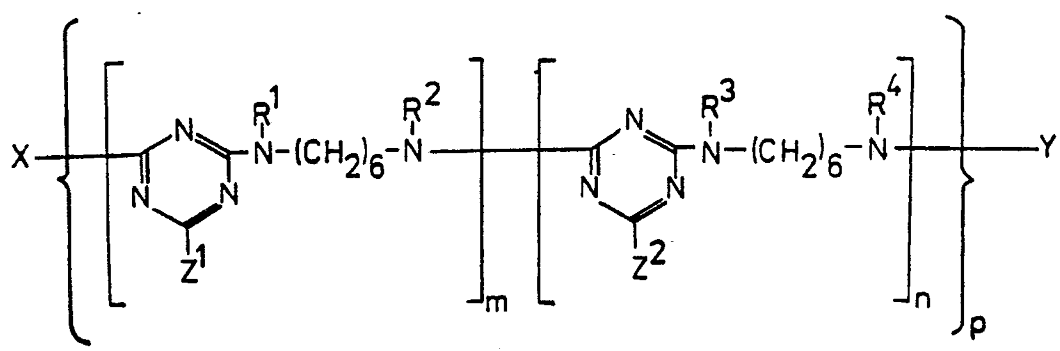

(I),

in which $R^1$ and $R^2$, either the same or different, stand for hydrogen or a group of the general formula II

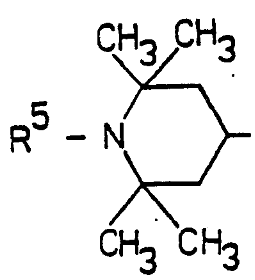

(II),

where $R^5$ stands for hydrogen or the methyl group, $R^3$ stands for hydrogen or group of the general formula III

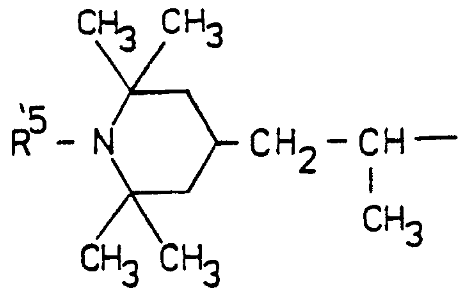

(III),

where $R^5$ has above meaning, $R^4$ is the group of the general formula III, and $Z^1$ and $Z^2$, either the same or different, stand for 2,2,6,6-tetramethyl-4-piperidylamino group, N(2,2,6,6-tetramethyl-4-piperidyl)$C_1$-$C_4$ alkylamino group, morpholino group, $C_1$-$C_{18}$-alkylamino group, di($C_1$-$C_4$ alkyl)amino group, cyclohexylamino group, dicyclohexylamino group, or N($C_1$-$C_4$ alkyl)cyclohexylamino group; X means chlorine, $C_1$-$C_{18}$ alkylamino group, 2,2,6,6-tetramethyl-4-piperidylamino group, cyclohexylamino group, morpholino group, a group of the general formula IV or a group of the general formula V

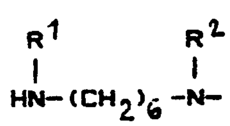

(IV);

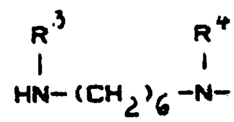

(V)

where $R^1$, $R^2$, $R^3$ and $R^4$ have the above meanings;
Y means hydrogen, a group of the formula VI or VI A

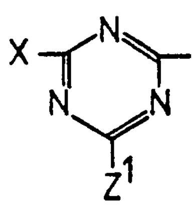

(VI),

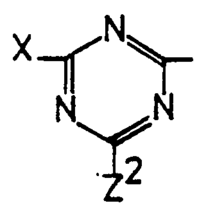

(VI A),

where X, $Z^1$, and $Z^2$ have the above meanings:
m is an integer from .1 to 10, n is an integer from 1 to 8 and p is a number from 1 to 5, the average molecular weight varying between 1500 and 12000.

The invention provides also a method for preparing substituted polyaminotriazines of the general formula I, wherein dichlorotriazine of the general formula VII and/or VII A

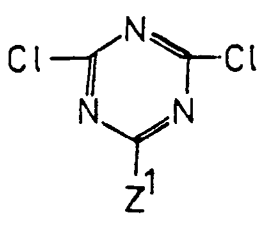

(VII),

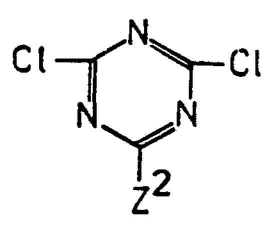

(VII A),

in which $Z^1$ and $Z^2$ have the above meanings, containing optionally, at the most 5 % by weight of cyanuric chloride as an accompanying component, is dissolved in an inert solvent, polycondensed in the presence of an inorganic base together with successively added diamines of the general formula VIII and IX

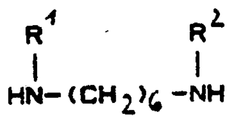 (VIII) 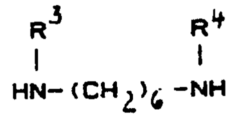 (IX)

where $R^1$, $R^2$, $R^3$, $R^4$ have the above meanings, at a temperature from 70 to 210 °C and under optional addition of an amino compound selected from the group comprising $C_1$-$C_{18}$ -alkylamine, 2,2,6,6-tetramethyl-4-aminopiperidine, cyclohexylamine and morpholine. The method can be modified by applying the following variants:

VARIANT A

Dichlorotriazine of the general formula VII and/or VII A containing at the most 5 wt % of cyanuric chloride as impurity is polycondensed with successively added diamines of the general formula VIII and IX in an inert organic solvent in the presence of an equivalent amount of the inorganic base, or its excess from 1 to 5 mol % relative to chlorine content in derivatives of dichlorotriazine and cyanuric chloride as accompanying component, for 4 to 20 hours at the temperature between 80 to 205 °C. The molar ratio of diamine VIII to diamine IX varies between 0.98 : 0.05 and 0.15 : 0.85, the total amount of dichlorotriazine VII and/or VII A being in a 1 to 25 mol % excess relative to the total molar amount of diamines VIII and IX. Chlorine remaining unchanged in the triazine units of the copolymer is eliminated to a value of up to 0.1 wt % by adding 5 to 35 mol % of amino compound relative to the dichlorotriazine derivatives used at 140-210 °C for 2 to 8 hours.

VARIANT B

In the first phase, dichlorotriazine VII containing at the most 5 wt % of cyanuric chloride as impurity is polycondensed together with diamine VIII in a molar ratio from 1:1.1 to 2, in an inert organic solvent, in the presence of an equivalent amount of an inorganic base with respect to the chlorine content in dichlorotriazine VII and cyanuric chloride, at 120-200 °C. In the second phase, dichlorotriazine VII or VII A is added, then diamine IX and an equimolar amount or 1 to 5 mol % excess of an inorganic base to dichlorotriazine are added either successively or at once, the polycordensation being finished at a temperature from 150 to 210 C. The total amount of diamines VIII and IX is in a 1 to 25 mol % excess relative to the total amount of dichlorotriazines of the general formula VII and/or VII A. It has been ascertained that it is advantagous to use toluene or xylene as inert solvents and sodium or potasium hydroxide as the inorganic base.

VARIANT C

Dichlorotriazine VII and/or VII A containing maximally 5 wt % of cyanuric chloride is gradually condensed in conjunction with components of the system comprising diamine VIII, diamine IX (0.9:1 to 0.15:0.85 molar ratio) and with an amino compound of 1 to 20 mol % concentration relative to the summary concentration of diamines in an inert organic solvent, in the presence of equivalent amount of an inorganic base when related to the chlorine content in derivatives of dichlorotriazine and cyanuric chloride, at a temperature from 120 to 210 °C for 4 to 15 hours. The total amount of diamines VIII and IX and the amino compound is in 1 to 20 mol % excess with respect to the summary amount of dichlorotriazine VII and/or VII A. It has been found that it is possible to use 1 to 5 mol % excess of the inorganic base with regard to the chlorine in the dichlorotriazine derivatives used.

When preparing polyaminotriazines according to the present invention aromatic hydrocarbons have been shown as inert organic solvent and inorganic bases such as alkaline metal hydroxides particularly sodium and potasium hydroxide as hydrogen chloride acceptor.

After the reaction has been finished, sodium or potasium chloride is separated by filtering, preferably after azeotropic distillation of water. By evaporating the solvent the final product is obtained from which low molecular weight substances can be separated by extraction with acetone containing a small amount of water, and then with acetone under optional cooling.

Dichlorotriazine VII and VII A are synthetized via reaction of primary or secondary amines with cyanuric

chloride in an equimolar ratio, in the presence of an inorganic base. As amine derivatives there have, for instance, been used: diethylamine, n-propylamine, isopropylamine, diisopropylamine, n-butylamine, di-n-butylamine, diisobutylamine, tert. butylamine, 2-amino-2,4,4-trimethylpentane, n-octylamine, n-dodecylamine, n-octadecylamine, morpholine, cyclohexylamine, dicyclohexylamine, N-methylaminocyclohexane, N-ethylaminocyclohexane, N-isopropylaminocyclohexane, 2,2,6,6-tetramethyl-4-aminopiperidene, 2,2,6,6-tetramethyl-4-(n-butylamino)-piperidine, 1-methyl-2--2-(2,2,6,6-tetramethyl-4-piperidyl)ethylamine (so far not used derivative). Substituted dichlorides of s-triazine can be used when they contain, as a contaminant, cyanuric chloride, 5 wt % at most. When purifying dichlorotriazine derivatives by distillation some problems with the solid phase cyanuric chloride (melting point 145 °C) in the distillation apparatus can to encountered.

Diamines VIII and IX are prepared by reduction alkylation of 2,2,6,6-tetramethyl-4-oxopiperidine, or 2,2,6,6-tetramethyl-4-piperidyleneacetone, using hexamethylenediamine and hydrogen in the presence of a metallic catalyst such as platinum or paladium. If using the corresponding derivative of piperidine and hexamethylenediamine (in 1:1 molar ratio) the desired 1-amino-6-(2,2,6,6-tetramethyl-4-piperidylamino)-hexane or not yet used 1-amino-6-[1-methyl-2-(2,2,6,6-tetramethyl-4-piperidyl) ethylamino]hexane are prepared, and at the 2:1 molar ratio di-substituted hexamethylenediamine derivatives are obtained which are represented by the desired 1,6-bis-(2,2,6,6-tetramethyl-4-piperidylaminohexane and so far not used 1,6-bis-[1-methyl-2-(2,2,6,6-tetramethyl-4-piperidyl)ethylamino]hexane.

Substituted polytriazines prepared according to the present invention show an excellent light stabilizing efficiency and can be processed without any problems together with thermoplastic polymers such as polyolefins, copolymers and homopolymers of styrene, polyethers, polyurethanes, polyesters, copolymers of butadiene, vinyl acetate and acrylonitrile. They exhibit a good compatibility to polymer matrices, are not volatile, show good heat stability, low migration and extractability from used polymers. They can preferably be applied to polyethylene and polypropylene, especially in film and fibre form. For the stabilization mixtures should be preferred containing from 0.05 to 3 wt % of such stabilizing agents, preferably within the range of from 0.1 to 0.7 wt %, and particularly in polymer systems containing antioxidants of phenolic, phosphite and other types, light stabilizing agens of 2-hydroxybenzophenone, benzotriazole and other types, combustion retarders, pigments, dyestuffs, various plasticizers, fillers, etc. Antioxidants are selected from the group of sterically hindered phenols comprising 2,6-di-tert.-butyl-4-methylphenol, 4,4-butylidene -bis-(2,6-di-tert.-butylphenol 4,4-thio-bis-(2-tert.butyl-5-methylphenol), phenolic derivatives of triazine compounds, thiodipropionic acid esters. From the group of phosphites dialklpentaerythrityldiphosphites, trinonylphosphite, triphenylphosphite and their combinations are suitable. 2-Hydroxy-4-alkoxybenzophenones, 2-(2-hydroxy-5-methylphenyl)benztriazol, octylphenylsalicylate, nickel-dithiocarbamate, nickel salts of 4-hydroxy-3,5-ditert.butylbenzylphosphonic acid may be selected from light stabilizers. Oligomeric copolymers according to the formula I can be added either in powder or granule form during mixing, heat treatment and processing of polymers, or during the preparation of prestabilized polymeric concentrates together with pigments, or the preparation of latex disperse systems.

The following examples are to be understood as ilustrative only, which means that they do not limit the scope of the present invention in any way.

## EXAMPLE 1

A copolymer with repeating units according to the general formula I, wherein $R^1$ and $R^2$ are 2,2,6,6-teteramethyl-4-piperidyl groups, $R^3$ and $R^4$ are 1-methyl-2-(2,2,6,6-tetramethyl-4-piperidyl)ethyl groups, Y is hydrogen, $Z^1$ and $Z^2$ are identical or stand for cyclohexylamino group, X is a group according to the general formula V, m = 3, n = 2, and average value p is 1.82, depending on the degree of conversion obtained and on the excess of diamino compound was prepared as follows:

Powdered NaOH (0.4 g) and 1.24 g (0.005 mol) of freshly redistilled 2,4-dichloro-6-cyclohexylamino-1,3,5-triazine in a solution of 5 ml xylene was added under stirring to a solution of 2.96 g (0.0075 mol) 1,6-bis-(2,2,6,6-tetramethyl-4-piperidylamino)hexane in 10 ml of xylene. Within one hour the reaction mixture was heated to the solvent reflux temperature and then annealed in a slight stream of nitrogen for 6 hours. After cooling the reaction mixture to 70 °C, 1.23 g 2,4-dichloro-6-cyclohexylamino-1,3,5-triazine containing 2.5 wt % cyanuric chloride (as impurities) in 5 ml xylene and 1.3 g of 35 % aqueous NaOH was added and then the reaction proceeded for another two hours at 70 C. Then 1.44 g (0.003 mol) 1,6-bis-[1-methyl-2-(2,2,6,6-tetramethyl-4-piperidyl)ethylamino ] hexane in a solution of 5 ml xylene was added and the reaction mixture was heated for 5 hours in an inert atmosphere under stirring at 195 C, and, after another addition of

0.1 g (0.0002 mol) of this diamine, the reaction was stopped after another two hours at 195 C. After distilling the azeotropic xylene/water mixture, sodium chloride was filtered off at 70 °C, a solid phase left on filter have been extracted with hot chloroform whereby losses of the product could be reduced. After evaporating the solvents, a yellowish product was obtained in the yield of 6.1 g (98 % theory), having an average molecular weight Mn = 3960 (VPO method) and the temperature interval of melting between 158 and 167 C. After repeated extraction of the oligomer with a small amount of acetone containing 5 vol. % of water, at 5 °C, the isolated product (5.7 g) had the temperature interval of melting from 162 to 171 °C and Mn = 4800.

## EXAMPLE 2

A copolymer of the general formula I, wherein $R^1$,$R^2$,$R^3$ and $R^4$ have the same meaningas in EXAMPLE 1, $Z^1$ is cyclohexylamino group, $Z^2$ is morpholino group, m = 9, n = 1 and average value of p is 1.01, Y is hydrogen and X is 2,2,6,6-tetramethyl-4-piperidylamino group, was prepared as follows:

To a solution of 3.552 g (0.009 mol) 1,6-bis-(2,2,6,6-tetramethyl-4-piperidylamino)hexane in 8 ml xylene 1.7 g of 50 % aqueous NaOH solution and 1.977 g (0.008 mol) 2,4-dichloro-6-cyclohexylamino-1,3,5-triazine in 8 ml xylene were added under stirring. The reaction mixture was heated for 5 hours at a reflux temperature, and, after cooling to 100 °C, there was at first added 0.247 g (0.001 mol) 2,4-dichloro-6-cyclohexylamino-1,3,5-triazine in 5 ml xylene and, after one hour, a mixture (heated to 100 C) of 0.479 g (0.001 mol) 1,6-bis 1-methyl-2-(2,2,6,6-tetramethyl-4-piperidyl)ethylamino hexane, 0.031 g (0.0002 mol) 2,2,6,6-tetramethyl-4-aminopiperidine and 0.235 g (0.001 mol) 2,4-dichloro-6-morpholino-1,3,5-triazine in 4 ml xylene was added. The reaction system was then heated under stirring in pressure reactor for 6 hours in an inert atmosphere at 195 °C and after another addition of 0.094 g (0.0006 mol) 2,2,6,6-tetramethyl-4-aminopiperidine, the reaction was stopped after two hours at 195 °C. The copolymer was isolated as describes in EXAMPLE 1. A yellowish product (175-183 °C melting point) obtained after extraction in the yield of 5.3 g (91 % theory) had an average molecular weight of 5980.

## EXAMPLE 3

A copolymer of the general formula I, wherein $R^1$, $R^2$, $R^3$ and $R^4$ have the same meaning as in EXAMPLE 1, $Z^1$ is cyclohexylamino group, $Z^2$ is n-octylamino group, m = 4, n = 1, average value p is 1.04, X is octadecylamino group, Y is a group of the general formula VI A in which X has the above meaning, was prepared as follows:

To a solution of 3.16 g (0.008 mol) 1,6-bis-(2,2,6,6-tetramethyl-4-piperidylamino)hexane in 10 ml xylene, 2.36 g of 50 % aqueos NaOH solution, 1.48 g (0.006 mol) 2,4-dichloro-6-cyclohexylamino-1,3,5-triazine in 5 ml xylene was added under stirring and after heating at reflux temperature another 0.49 g (0.002 mol) of this dichloride in 2 ml xylene was added. The reaction system was then cooled to 100 °C and then a solution (heated to 100 °C) of 0.69 g (0.0025 mol) 2,4-dichloro-6-octylamino-1,3,5-triazine and 0.96 g (0.002 mol) 1,6-bis- [ 1-methyl--2-(2,2,6,6-tetramethyl-4-piperidyl)ethylamino]hexane in 5 ml xylene was added, and the reaction mixture was heated under stirring for 5 hours at 200 C in an inert atmosphere in a pressure reactor. After cooling to 100 C, 0.39 g (0.0014 mol) 2,4-dichloro-6-octylamino-1,3,5-triazine was added and after one hour 0.94 g (0.0035 mol) octadecylamine was added and the mixture was heated under stirring at 200 °C for 3 hours in pressure reactor. After the reaction, water was separated from the mixture in the form of an azeotropic mixture with the solvent. The product was isolated as described in EXAMPLE 1. The yellowish product obtained after extraction at 6.7 g (92 %) yield had the temperature interval of melting between 148 and 157 °C, and its molecular weight determined by VPO method was 3860. The oligomer did not contain chlorine.

## EXAMPLE 4

A copolymer of the general formula I, wherein $R^1$ and $R^2$ are 1,2,2,6,6-pentamethyl-4-piperidyl groups, $R^3$ and $R^4$ are 1-methyl-2-(1,2,2,6,6-pentamethyl-4-piperidyl)ethyl groups, $Z^1$ is N(ethyl)cyclohexylamino group, $Z^2$ is N(methyl)-1,2,2,6,6-penta methyl-4-piperidylamino group, m = 4, n = 1, average value p is 1.01 Y

is methyl, and X is morpholino group, was prepared from an intermediate product by methylation of -NH groups as follows:

To a solution containing 9.472 g (0.024 mol) 1,6-bis-(2,2,6,6-tetramethyl-4-piperidylamino)hexane, 4.95 g (0.018 mol) 2,4-dichloro-6-N(ethyl)cyclohexylamino-1,3,5-triazine in 30 ml toluene 5.1 g of 50 % aqueous NaOH solution was added, and after 5 hours heating under reflux another 1.65 g (0.006 mol) of this dichloride in 5 ml toluene was added. Then a solution heated to 80 ° C and containing a reaction system of 2.87 g (0.006 mol) 1,6-bis- [ 1-methyl-2(2,2,6,6-tetramethyl-4-piperidyl)ethylamino] hexane, 0.13 g (0.0015 mol) morpholine and 1.83 g (0.006 mol) 2,4-dichloro-6-(2,2,6,6-tetramethyl-4-piperidylamino)-1,3,5-triazine in 15 ml toluene was added. The reaction mixture was heated for 15 hours at 190 ° C in pressure reactor under inert atmosphere, and after adding 0.37 g (0.0042 mol) morpholine the reaction was stopped after 2 hours at 190 ° C. The product isolated as described in EXAMPLE 1 (18.6 g yield, 97.5 %) had the temperature interval of melting from 155 to 165 ° C and Mn 3190. After extracting 15.6 wt % of a fraction having Mn 2050, the product had Mn 3650 and the temperature interval of melting 160-172 ° C. Another modification of the oligomer was carried out by methylation of secondary amino groups of piperidine derivatives, cyclohexylamino groups, and final groups of diamino derivatives as follows:
The copolymer prepared (7.5 g) was added to a solution containing 4.5 g formic acid (90 wt % conc.), 7.5 g aqueous formaldehyde solution (35 wt % conc.), and 6 ml water. The mixture was heated under stirring and reflux for 15 hours. A viscous product, once cooled, was diluted with 60 ml water and then precipitated by adding 5 wt % NaOH aqueous solution. The product separated by filtration, after repeated washing with water and drying, had the melting temperature from 188 to 198 ° C and Mn 3980.

## EXAMPLE 5

A copolymer of the general formula I, wherein $R^1$ is hydrogen or 2,2,6,6-tetramethyl-4-piperidyl group, $R^2$, $R^3$ and $R^4$ have the same meaning as in EXAMPLE,1, $Z^1$ is N(methyl)cyclohexylamino group, $Z^2$ is dicyclohexylamino group, X is chlorine or group of the formula V, m = 3, n = 1 and the average value of p is 1.32, was prepared as follows:

To a solution of 1.973 g (0.005 mol) 1,6-bis(2,2,6,6-tetramethyl-4-piperidylamino)hexane and 0.639 g (0.0025 mol) 1-amino-6-(2,2,6,6-tetramethyl-4-piperidylamino)hexane in 10 ml xylene 0.42 g powdered NaOH and 1.306 g (0.005 mol) 2,4-di-chloro-6-N(methyl)cyclohexylamino-1,3,5-triazine in 5 ml xylene was added. The reaction mixture was heated to the boiling temperature of the solvent and kept at this temperature for 3 hours under stirring. Then 0.652 g (0.0025 mol) 2,4-dichloro-6-N(methyl)cyclohexylamino-1,3,5-triazine in 5 ml xylene was added to the reaction mixture. After one hour reflux heating, 1.3 g of 35 % aqueous NaOH solution were added at first and then a reaction mixture was heated to 130 C and 0.823 g (0.0025 mol) 2,4-dichloro-6-dicyclohexylamino-1,3,5-triazine and 1.437 g (0.003 mol) 1,6-bis[1-methyl-2-(2,2,6,6-tetramethyl-4-piperidyl) ethylamino]hexane in 5 ml xylene was added under stirring. The polycondensation is finished in a pressure reactor by heating at 190 ° C for 6 hours. After separating the inorganic component and the solvent 5.9 g (96 %) of a yellowish product (Mn 3300, the temperature interval of melting 155-160 ° C) was obtained.

## EXAMPLE 6

A copolymer of the formula I, where $R^1$, $R^2$, $R^3$ and $R^4$ have the same meaning as in EXAMPLE 1, $Z^1$ is cyclohexylamino group, $Z^2$ is 1-methyl-2-(2,2,6,6-tetramethyl-4-piperidyl)ethylamino group,m = 4 n = 1, average value p is 1.1, X is 2,2,6,6-ttetramethyl-4-piperidylamino group, and Y is group of the formula VI A, was prepared as follows:

To a solution of 3.158 g (0.008 mol) 1,6-bis-(2,2,6,6-tetramethyl-4-piperidylamino)hexane in 10 ml xylene 0.85 g of powdered NaOH and a solution containing 1.483 g (0.006 mol) 2,4-dichloro-6-cyclohexylamino-1,3,5-triazine in 5 ml xylene were added. Within one hour, the mixture was heated under stirring to a temperature of solvent reflux, and, after an another 5 hour heating, another 0.949 g (0.002 mol) of this dichloride in 5 ml xylene was added. Then the reaction mixture was heated to 130 ° C and 0.958 g (0.002 mol) 1,6-bis-[1-methyl-2(2,2,6,6-tetramethyl-4-piperidyl)ethylamino ] hexane and 0.863 g (0.0025 mol) 2,4-dichloro-6-[1-methyl-2-(2,2,6,6-tetramethyl-4-piperidyl-amino)]-1,3,5-triazine in 5 ml xylene is added and after adding 0.8 ml water, the reaction system was heated for 4 hours at 195 C in pressure reactor; after

another addition of 0.45 g (0.0013 mol) of this dichloro derivative, the reaction continued for one hour at 195 ° C. To eliminate unreacted chlorine groups, after cooling the system, 0.55 g (0.0035 mol) 2,2,6,6-tetramethyl-4-aminopiperidine was added. The mixture was heated under mixing in pressure reactor in an inert atmosphere at 195 ° C for 8 hours. The product was finally isolated as reffered to in EXAMPLE 1. The yellowish product obtained after extraction (5.9 9, 96 %) had melting temperature between 152 and 164 ° C, and Mn 3890. The product did not contain chlorine.

EXAMPLE 7

A copolymer of the formula I, wherein $R^1 = R^2 = H$, or stand for 2,2,6,6-tetramethyl-4-piperidyl groups, $R^3$ is hydrogen, $R^4$ is 1-methyl-2-(2,2,6,6-tetramethyl-4-piperidyl)ethyl group, $Z^1$ is cyclohexylamino group, $Z^2$ is diethylamino group, X is group of the formula V, m = 3, n = 1, and average value p is 1.38, was prepared as follows:

To a solution containing 1.973 g (0.005 mol) 1,6-bis-(2,2,6,6-tetramethyl-4-piperidylamino)hexane and 0.291 g (0.0025 mol) hexamethylenediamine in 10 ml xylene 1.236 g (0.005 mol) 2,4-dichloro-6-cyclohexylamino-1,3,5-triazine in 5 ml xylene, and 0.42 g (0.0105 mol) of powdered NaOH were added. The reaction mixture was heated to the boiling temperature of the solvent within one hour and kept at this temperature under stirring for another 5 hours. Then additional 0.618 g (0.0025 mol) of this dichloride was added, and after one hour a mixture (heated to 130 ° C) was added comprising 0.553g (0.0025 mol) 2,4-dichloro-6-diethylamino-1,3,5-triazine and 0.693 g (0.003 mol) 1-amino-6-[1-methyl-2-(2,2,6,6-tetramethyl-4-piperidyl)ethylamino] hexane in 5 ml xylene. After adding 1.3 g of 35 % aqueous NaOH the reaction mixture was heated under inert atmosphere in pressure reactor at 200 ° C under stirring for 5 hours, and after another addition of 0.1 g (0.0002 mol) of this diamine, the reaction was stopped after 2 hours at 200 ° C. After the isolation as described in EXAMPLE 1 a yellowish product (4.6 g, 95 %, melting temperature between 158-166 ° C, Mn 2880) was obtained.

EXAMPLE 8

A copolymer of the general formula 1, wherein groups $R^1$. $R^2$, $R^3$ and $R^4$, $Z^1 = Z^2$ and Y have the same meaning as in EXAMPLE 1, X is group of the general formula IV, m = 1, n = 1 and average value p is 1.02, was prepared as described in EXAMPLE 1 by using 3.591 g (0.0075 mol) 1,6-bis-[1-methyl-2-(2,2,6,6-tetramethyl-4-piperidyl)ethylamino] hexane and 2.471 g (0.01 mol) 2,4-dichloro-6-cyclohexylamino-1,3,5-triazine in solution of 10 ml xylene, and by adding 0.85 g powdered NaOH and 1.184 g (0.003 mol) 1,6-bis-(2,2,6,6-tetramethyl-4-piperidylamino)hexane in 5 ml xylene. After adding all of the above components, the reaction mixture was heated for 5 hours at 195 ° C. Unreacted -Cl groups were eliminated by another addition of 0.79 g (0.002 mol) diamine and by heating for 2 hours at 195 ° C. A brownish product (6.9 g, 94 %) had a melting temperature between 92 and 103 ° C, and molecular weight Mn 2730.

EXAMPLE 9

Polyaminotriazine was prepared according to EXAMPLE 2, except for that to a solution containing 3.552 g (0.009 mol) of 1,6 -bis ( 2,2,6,6 - tetramethyl -4 -piperidyl amino) hexane, 0.479 g (0.001 mol) of 1,6 - bis - /1-methyl -2- (2,2,6,6-tetramethyl- 4 -piperidyl) ethylamino/ hexane, 0.063 g (0.0004 mol) of 2,2,6,6 - tetramethyl - 4 -aminopiperidine in 8 ml of toluene was under stirring added to 1.7 g of 50% w. of aqueous solution of NaOH and to a solution of 1.978 g (0.008 mol) of 2,4 - dichloro - 6 -cyclohexylamino - 1,3,5 - triazine and 0.47 g (0.002 mol) of 2,4 - dichloro - 6 - morpholino - 1,3, 5 -triazine in 8 ml of toluene. The reaction system was kept in a pressure reactor at 205 ° C for 8 hours under stirring. Yelowish product (5.7 g; 98% of theory) which was isolated according to EXAMPLE 1, had the molecular mass Mn = 3300 and melted within 169 and 181 ° C.

EXAMPLE 10

Polyaminotriazine was prepared according to EXAMPLE 3, except for that a solution containing 3.16 g (0.008 mol) of 1,6 - bis -(2,2,6,6 - tetramethyl - 4 - piperidylamino) hexane and 0. 96 g (0.002 mol) of 1.6 - bis - /1 - methyl - 2 - (2,2,6,6 -tetramethyl - 4 - piperidyl) ethylamino/ hexane in 8 ml of toluene was added to 1.7 g of 50 wt % of aqueous solution of NaOH and, under permanent stirring, to a solution of 1.24 g (0.0085 mol) of 2,4 - dichloro - 6 - cyclohexylamino - 1,3,5 -triazine and 1.52 g (0.0055 mol) of 2,4 - dichloro - 6 - (1,1,3, 3 - tetramethyl) butylamino - 1,3,5 - triazine in 8 ml of toluene. The polycondensation reaction was performed in a pressure reactor at 265° C for 6 hours under stirring. After cooling, unreacted Cl- groups were removed by addition 0.26 g (0.003 mol) of morpholine and the system was kept in the reactor at 190° C for further 5 hours. The product isolated according to EXAMPLE 1 had the molecular weight Mn 3260 and melted within the range 146 and 156° C.

## EXAMPLE 11

A copolymer was prepared according to EXAMPLE 7, except for that a solution of 1.97 g (0.005 mol) of 1,6 - bis - (2,2,6,6 -tetramethyl - 4 - piperidylamino) hexane, 0.29 g (0.0025 mol) of hexamethylene diamine and 1.44 g (0.003 mol) of 1,6 - bis - /1 - methyl - 2 - (2,2,6,6 - tetramethyl - 4 - piperidyl) ethylamino/ hexane in 5 ml of toluene was added to 1.7 g of aqueous solution of NaOH and then, under permanent stirring, to a solution of 1.24 g (0.005 mol) of 2,4 - dichloro - 6 -cyclohexylamino -1,3,5 - triazine, 0.62 g (0.0025 mol) of 2,4 - dichloro - 6 -diisopropylamino - 1,3,5 - triazine and to 0.69 g (0.0025 mol) of 2,4 - dichloro - 6 - (1,1,3,3 - tetramethyl) butylamino -1,3,5 - triazine in 5 ml of toluene. The reaction mixture was kept at 205 C in a pressure reactor under stirring at 205° C for 8 hours. The product isolated according to EXAMPLE 1 (5.4 g; 97.8 % of theory) had molecular weight Mn 2850 and melted within the range 123 and 134° C.

## EXAMPLE 12

A copolymer was prepared according to EXAMPLE 1, except for that 2.96 g (0.0075 mol) of 1,6 - bis - (2,2,6,6 - tetramethyl -4 - piperidylamino) hexane and 1.44 g (0.003 mol) of 1,6 - bis /1 - methyl - 2 - (2,2,6,6 - tetramethyl - 4 - piperidyl) ethylamino/ hexane in 5 ml of toluene were added to 1.7 g of 50 wt. % of aqueous solution of NaOH and then, under stirring, to the solution of 2.42 g (0.0098 mol) of 2,4 - dichloro - 6 -cyclohexylamino - 1,3,5 - triazine, 0.03 g (0.00017 mol) of cyanuric chloride in 5 ml of toluene. The reaction system was kept at 195 C in a pressure reactor under stirring for 10 hours. After isolation, 5.9 g (96.2 % of theory) of the product was obtained of molecular weight Mn 3540, which melted within 153 and 161° C.

## EXAMPLES 13 TO 16

Copolymers were prepared according to EXAMPLE 3, except for that 3.16 g (0.008 mol) of 1,6 - bis - (2,2,6,6 - tetramethyl -4 - piperidylamino) hexane, 0.38 g (0.0008 mol) of 1,6 - bis -/1 - methyl - 2 - (2,2,6,6 - tetramethyl - 4 - piperidyl) ethylamino/ hexane and 0.09 g (0.0008 mol) of hexamethylene diamine in 5 ml of toluene were added to 1.7 g of 50 wt. % of aqueous solution of NaOH and then, under stirring, to a solution of 0.055 g (0.0003 mol) of cyanuric acid, 1.48 g (0.006 mol) of 2,4 - dichloro - 6 - cyclohexylamino - 1,3,5 - triazine and to 0.004 mol of substituted 2,4 - dichloro - s - triazine (VII A -substituent is denoted by Z ) dissolved in 5 ml of toluene (See TABLE 1). The reaction mixture was kept at 205 °C for 5 hours, then cooled and 0.01 mol of amino compound was added (See TABLE 1) and unreacted Cl- groups were removed by further reaction at 205° C for 4 hours. Molecular weights and temperature intervals of melting of products are summarized in TABLE 1.

Table 1

| Example No. | VII A (0.004 mol) group $Z^2$ = | Amino compound | Temperature interval of melting, °C | Mn |
|---|---|---|---|---|
| 13 | N(ethyl)-cyclohexylamino | 2,2,6,6-tetramethyl-4-amino piperidine | 162-170 | 2190 |
| 14 | N(Isopropyl)cyclohexylamino | tert.butyl amine | 167-176 | 2710 |
| 15 | dicyclohexylamino | octadecylamine | 147-155 | 1970 |
| 16 | diethylamino | morpholine | 158-167 | 2638 |

EXAMPLES 17 and 18

Copolymers were prepared according to EXAMPLE 3, except for that 0.479 g (0.001 mol) of 1,6 - bis - /1 - methyl - 2 -(2,2,6,6 - tetramethyl - 4 - piperidyl) ethylamino/ hexane, 0.275 g (0.001 mol) of 2,4 - dichloro - 6 - (N - ethyl) cyclohexylamino - 1,3,5 -triazine in 4 ml of toluene were added at 70 °C to 1.7 g of 50 wt. % aqueous solution of NaOH and, under stirring, to 3.552 g (0.009 mol) of 1,6 - bis - (2,2,6,6 -tetramethyl - 4 - piperidylamino) hexane and 2.633 g (0.0095 mol) of 2,4 - dichloro - 6 - octylamino - 1,3,5 - triazine (DOAT) or 2.233 g (0.0095 mol) of 2,4 - dichloro - 6 -morpholino - 1,3,5 - triazine (DCMT) in 6 ml of toluene (See TABLE 2). The reaction mixture was kept at 195 °C for 5 hours, then cooled, and unreacted Cl- groups were removed by reaction with 0.01 mol of amino compound (See TABLE 2) carried out at 95 °C for 4 hours. The yield, molecular weight and temperature interval of melting of products obtained for variable reaction components are given in TABLE 2.

Table 2

| Example No. | Dichloride (0.0095 mol) | Amino compound | Yield g | Temperature interval of melting °C | Mn |
|---|---|---|---|---|---|
| 17 | DOAT | cyclohexylamine | 1.0 | 142-155 | 3650 |
| 18 | DCMT | octylamine | 1.3 | 168-180 | 2920 |

EXAMPLE 19

Blends of polypropylene (unstabilized powder of HPF polypropylene, flow index FI (230 °C) = 8 g/10 minutes) containing wt. % of 2,6 -ditert.butyl - 4 - methyl phenol, 0.15 % w. of calcium stearate, 0.05 % w. of tris - (3,5 -ditert.butyl - 4 - hydroxybenzyl) isocyanate and 0.3 % w. of stabilizing agent tested were prepared and homogenized in a chamber of plastograph Brabender at 190 °C for 10 minutes and under nitrogen atmosphere. The foils 0.1 mm thick were obtained by pressing at 260 °C. Reference foils of pure polypropylene and those with additives without a light stabilizer prepared in the same way were also examined. The photostabilizing efficiency of products prepared according to EXAMPLES 1,3,5,6,10 and 12 in polypropylene has been compared with the efficiency of the commercial light stabilizer poly </tert.octylamino - 1,3,5 -triazine - 2,4 - diyl/ - /2 - (2,2,6,6 - tetramethyl - 4 -piperidyl) imino/ - hexamethylene> (European patent 93693) which contained 0.3 wt. % of residual chlorine. The polymer foils were exposed to photo-oxidation in Xenotest 1200 (Original Hanau 1200, FRG) and an increment of carbonyl groups band ($\Delta A_{CO}$) has been determined with IR spectrometer. The stabilizing efficiency was

expressed in hours of photo-oxidation corresponding to the increase of absorbance of CO absorbtion band by 0.3 ($\Delta A_{CO}$ = 0.3). The values found are in TABLE 3.

Table 3

| Tested system | Photo-oxidative stability Xenotest 1200 ($\Delta A_{CO} = 0.3$), hours |
|---|---|
| Pure unstabilized polypropylene | 200 |
| Polypropylene with additives without a light stabilizer | 400 |
| Polypropylene with product from Example 1 | 1750 |
| Example 3 | 1830 |
| Example 5 | 1780 |
| Example 6 | 1920 |
| Example 10 | 1780 |
| Example 12 | 1950 |
| Polypropylene with a light stabilizer prepared according to European Patent No 93 693 | 1750 |

## EXAMPLE 20

The polypropylene foils 0.1 mm thick, prepared by pressing of 0.5 mm thick foils at 200 °C, corresponding to blends from EXAMPLE 19, were exposed to thermo-oxidative stability tests in drying oven kept at 110 °C. The results of the test are summarized in TABLE 4.

Table 4

| Tested system | Thermo-oxidative stability, h |
|---|---|
| Pure unstabilized polypropylene | 40 |
| Polypropylene with additives except for a light stabilizer | 120 |
| Polypropylene with a product from Example 1 | 1600 |
| Example 4 | 1800 |
| Example 6 | 2050 |
| Example 7 | 1850 |
| Example 8 | 1700 |
| Example 10 | 1820 |
| Example 12 | 1950 |
| Polypropylene with a light stabilizer prepared according to European Patent No. 93693 | 1600 |

## EXAMPLE 21

Unstabilized polypropylene powder (polypropylene HPF, flow index FI (230 °C) = 11.2/10 minutes) was blended with 0.1 wt. % of tris - (3,5 - ditert.butyl - 4 - methyl phenol, 0.05 wt. % of tris - (3,5 - ditert.butyl - 4 - hydroxybenzyl) isocyanurate, 0. 15 wt. % of calcium stearate and 0.2 wt. % of a copolymer prepared aocording to this invention. The granules of the polymer system prepared in an extruder at 230 °C were spinned in a device with the screw-feeding machine of diameter 32 mm. Stabilized fibres obtained for the polymer feeding 23 g/min, for the temperatures of the thermostatted zone 260 °C and of extruder nozzle 270 °C (type 20/0.5) with the speed of winding up 600 m/min were stretched at 120 °C to finishing stretch ratio 1:3.5. The fibers had fineness 124 dtex, strength 436 cN (3.57 cN/dtex) and elongation at break 42 %. They were subjected to exposition in Xenotest 450 (Original Hanau, FRG), until the tensile strength was reduced to 50 % of its original value. The corresponding times ($t_{50}$) are given in TABLE 5.

Table 5

| Stabilizer | Xenotest 450 $t_{50}$ , h |
|---|---|
| Product according to | |
| Example 1 | 1160 |
| Example 2 | 920 |
| Example 7 | 980 |
| Example 9 | 960 |
| Example 11 | 940 |
| Example 17 | 1100 |
| Light stabilizer prepared according to European Patent No. 93 693 | 1020 |

## EXAMPLE 22

Unstabilized low density polyethylene of flow index IT (190 °C) = 2 g/10 min was blended with 0.05-0.5 wt. % of stabilizer examined and with 0.1 wt. % of calcium stearate (plastograph Brabender, 160 °C, nitrogen, 10 minutes). Foils 0.5 mm thick were then pressed at 180 °C. Foils were exposed to irradiation in Xenotest 450. Their photo-oxidation stability was estimated from the time in hours necessary for obtaining the increment in the absorbance of CO bands 0.5 (TABLE 6).

Table 6

| Stabilizer | w. % | time of exposure for $\Delta A_{CO}$ = 0.5, h |
|---|---|---|
| - | 0 | 1700 |
| Product from Example 3 | 0.2 | 3550 |
| Example 3 | 0.3 | 4150 |
| Example 7 | 0.05 | 1850 |
| Example 11 | 0.1 | 2900 |
| Example 11 | 0.2 | 3700 |
| Example 15 | 0.2 | 3650 |
| Example 15 | 0.5 | 5500 |
| Example 18 | 0.2 | 3500 |
| Example 18 | 0.5 | 6000 |
| Light stabilizer according to European patent No. 93 693 | 0.2 | 3450 |
| | 0.3 | 4000 |

## EXAMPLE 23

Unstabilized high density polyethylene (flow index FI (190 °C) = 6 g/10 min) was mixed with 0.05 wt. % of 2,6 - ditert.butyl - 4 - methyl phenol, 0.05 - 2 wt. % of the stabilizer tested and with 0.1 wt. % of calcium stearate (plastograph Brabender, nitrogen, 175 °C, 10 minutes). From prepared mixtures the foils 0.1 mm thick were then pressed at 200 °C. The foils were exposed in a Xenotest 450 until the absorbance of CO band has increased by 0.1. The corresponding times in hours are in TABLE 7.

Table 7

| System | % w. | time of exposure for $\Delta A = 0.1$, h |
|---|---|---|
| Pure unstabilized polyethylene | - | 800 |
| Polyethylene without light stabilizer | - | 1100 |
| Polyethylene with product | | |
| from Example 1 | 0.2 | 5300 |
| Example 6 | 0.05 | 1800 |
| Example 6 | 0.2 | 4950 |
| Example 9 | 0.2 | 4850 |
| Example 9 | 0.5 | 6800 |
| Example 11 | 0.2 | 4050 |
| Example 11 | 2.0 | 10000 |
| Example 16 | 0.2 | 4880 |
| Example 16 | 1.0 | 7500 |
| Polyethylene with light stabilizer according to European patent No. 93 693 | 0.2 | 4700 |

EXAMPLE 24

Unstabilized high molecular weight polyethylene (flow index FI (190 °C) = 0.14 g/10 min) was mixed with 0.2-0.5 wt. % of stabilizer tested and 0.1 wt. % of calcium stearate (plastograph Brabender, 190 °C, nitrogen, 10 minutes). From the mixtures prepared in this way, foils 0.1 mm thick were pressed at 220 °C. The foils were thermostatted in drying oven and exposed in a Xenotest 450 until the absorbance of carbonyl groups band increased by 0.1 in the drying oven and 0.3 in Xenotest 450. The times necessary to achieve these values are given in TABLE 8

Table 8

| System | wt. % | drying oven 100 °C time for $\Delta A_{CO}$ = 0.1 h | Xenotest time for $\Delta A_{CO}$ = 0.3 h |
|---|---|---|---|
| Polyethylene without stabilizer | 0 | 150 | 600 |
| Polyethylene with product from Example 3 | 0.2 | 2800 | 5600 |
| Example 13 | 0.2 | 3050 | 5750 |
| Example 13 | 0.5 | 5000 | 8000 |
| Example 17 | 0.2 | 2900 | 5550 |
| Example 18 | 0.5 | 5000 | 8000 |
| Polyethylene with light stabilizer according to European patent No. 93 693 | 0.2 | 2750 | 5200 |

## EXAMPLE 25

Unstabilized low density polyethylene (flow index FI (190 °C) = 1.4 g/10 minutes) was blended with 0.05-1.0 wt. % of produced stabilizers. The mixtures were processed in an extruder (Werner Pfleiderer ZSN) at the temperatures 165,175,180,185 and 190 °C at the screw speed 200 rpm to granules which were then pressed to foils 0.1 mm thick using the equipment Buzuluk (diameter 32 mm). The temperature varied from 165 to 185 °C, the screw speed was 100 rpm. The foils were exposed in a Xenotest 450 and the time (in hours) necessary to obtain an increment in the absorbance of CO bands 1.0. The results of the test are in TABLE 9.

Table 9

| System | wt. % | time of exposure for A = 1.0, h |
|---|---|---|
| Polyethylene without stabilizer | 0 | 1500 |
| Polyethylene with the product from Example 3 | 0.05 | 2200 |
| Example 12 | 0.2 | 4400 |
| Example 12 | 1.0 | 8000 |
| Example 14 | 0.2 | 4650 |
| Example 14 | 0.5 | 6800 |

## EXAMPLE 26

Unstabilized ethylene/vinyl acetate (EVA) copolymer (5 wt. % of vinyl acetate, flow index FI (150 °C) = 2 g/10 min) was mixed with 0.2-0.3 wt. % of a stabilizer prepared. The blends were processed in a Werner-Pfleiderer extruder to granules at the temperature from 175 to 195 °C and at 100 rpm; the granules were pressed to foils 0.08 mm thick (equipment Buzuluk, diameter of screw 32 mm, temperature from 175 to 195 °C, the screw speed 120 rpm). The light stability of foils towards Xenotest 1200 exposure and towards natural atmospheric weathering has been determined. The stability was expressed by the time necessary for reduction of elongation at break to 50 % of its original value ($t_{50}$). The results are in TABLE 10.

Table 10

| System | wt. % | Xenotest $t_{50}$ ,h | Weathering $t_{50}$,months |
|---|---|---|---|
| EVA copolymer without stabilizer | 0 | 760 | 2.5 |
| EVA copolymer with product of Example 3 | 0.2 | 1100 | 6 |
| Example 16 | 0.2 | 1250 | 7.5 |
| Example 16 | 0.3 | 1600 | 9.5 |
| Example 18 | 0.2 | 1200 | 8 |
| EVA copolymer with poly-(N-2-hydroxyethyl-2,2,6,6-tetramethyl-4-hydroxypiperidyl) succinate | 0.2 | 860 | 4.5 |

## EXAMPLE 27

0.5 wt. % of a stabilizer prepared was added to the solution of polystyrene (5 % w.) in chloroform (Flow index of polystyrene FI (200 °C) = 16.5 g/10 min.) and the stabilizer was dissolved. The solution was poured on Petri dishes. Foils 0.05 mm thick were obtained by a free evaporation of chloroform. The foils were exposed in a Xenotest 450 until the complete degradation has been achieved. The corresponding times are given in TABLE 11.

Table 11

| System | wt. % | Xenotest 450, h |
|---|---|---|
| Polystyrene without stabilizer | 0 | 500 |
| Polystyrene with the product from Example 6 | 0.5 | 1300 |
| Example 12 | 0.5 | 1500 |
| Example 14 | 0.5 | 1400 |
| Example 15 | 0.5 | 1550 |
| Example 18 | 0.5 | 1470 |

## EXAMPLE 28

ABS copolymer (flow index FI (220 °C) = 1812 g/10 min.) was mixed with 5 wt. % of a stabilizer and foils 0.1 mm thick were prepared. The foils were irradiated by a 400 W Hg-lamp provided with a Pyrex filter. The induction period (h) corresponds to the ratio A 1745/A 1940 determined from IR spectroscopy. The results of test are in TABLE 12.

Table 12

| System | hours |
|---|---|
| ABS copolymer without a stabilizer | 30 |
| ABS copolymer with the product from Example 2 | 80 |
| Example 3 | 100 |
| Example 11 | 95 |
| ABS copolymer with 2-(2-hydroxy-5-methylphenyl) benzotriazole | 60 |

## EXAMPLE 29

Unstabilized polycarbonate (flow index FI (300 °C) = 8.6 g/10 min) was blended with 0.1 wt. % of 3,5-ditert.butyl - 4 -hydroxy phenyloctadecylpropionate, 0.1 wt. % of tris - (2,4 -tert.butyl phenyl) phosphite and 0.3 wtm % of a light stabilizer prepared. The blends were processed in a Werner-Pfleiderer extruder at 245, 275, 258, 226 and 256 °C at 187 rpm and pressed to foils 0.5 mm thick. The index of yellowing (IY) has been determined through the relationship

$$IY = \frac{\Delta T_{(420)} - \Delta T_{(480)}}{T_{560}} \cdot 100$$

where $\Delta T$ is a loss of transmittance, corresponding to respective wavelengths, following the irradiation of samples in Xenotest, and $T_{560}$ is the value of transmittance (%) of nonirradiated sample. The results are in TABLE 13.

Table 13

| System | IY after irradiation in Xenotest 450 | |
|---|---|---|
| | after 3000 h | after 9000 h |
| Polycarbonate without a light stabilizer | 5.2 | 11.0 |
| Polycarbonate with 2-(2-hydroxy-5-methylphenyl) benzotriazole | 2.5 | 5.8 |
| Polycarbonate with the product from Example 6 | 2.2 | 5.3 |
| Example 7 | 2.1 | 5.0 |
| Example 13 | 2.3 | 5.5 |
| Example 17 | 2.2 | 5.2 |

## Claims

1. Substituted polyaminotriazines of the general formula I

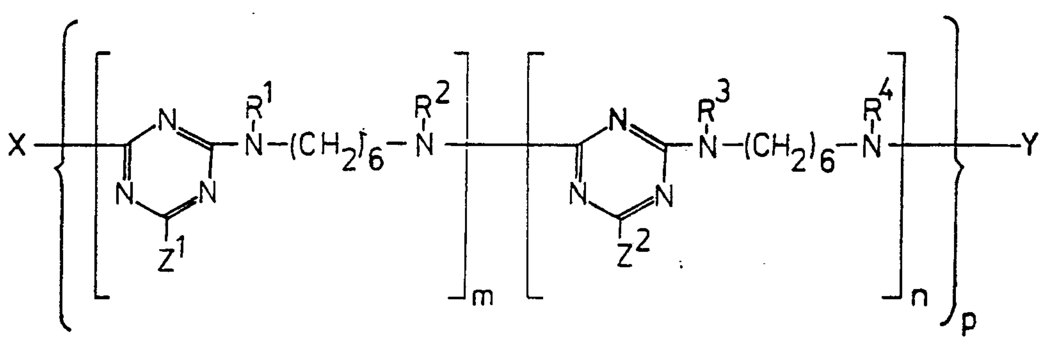

(I),

in which $R^1$ and $R^2$, either the same or different, stand for hydrogen or a group of the general formula II

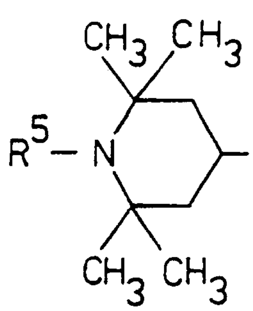

(II),

where $R^5$ stands for hydrogen and methyl group, $R^3$ stands for hydrogen or a group of the general formula

III

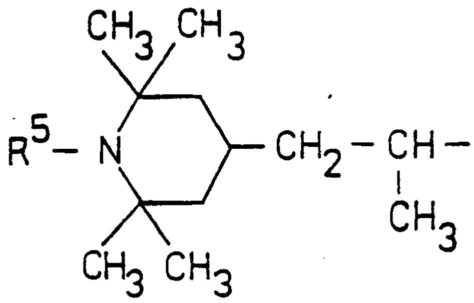

(III),

where $R^5$ has the above meaning,

$R^4$ stands for a group of the general formula III,

$Z^1$ and $Z^2$, either identical or different, stand for 2,2,6,6 -tetramethyl - 4 - piperidylamino group, morpholino group, $C_1$- $C_{18}$ alkylamino group, di ($C_1$ - $C_4$ ) alkylamino group, dicyclohexylamino group or N ($C_1$ - $C_4$alkyl) - cyclohexylamino group,

X stands for chlorine, $C_1$- $C_{18}$ alkylamino group, 2,2,6,6 -tetramethyl - 4 - piperidylamino group, cyclohexylamino group, group of the general formula IV or V

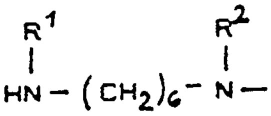

IV)

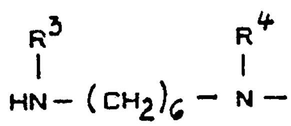

V)

where $R^1$, $R^2$, $R^3$ and $R^4$ have the above meanings.

Y stands for hydrogen or a group of the general formula VI or VI A

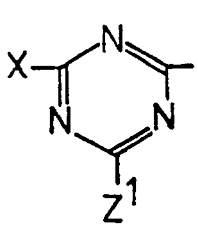

(VI),

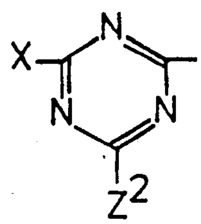

(VI A),

where X, $Z^1$ and $Z^2$ have the above meanings,

m is an integer between 1 and 10,

n is an integer between 1 and 8,

p is a number between 1 and 5.

Molecular weight Mn of products varies between 1500 and 12000.

2. A method of synthesis of substituted polyaminotriazines as claimed in claim 1 characterised by the polycondensation of dichlorotriazine of the general formula VII and VII A, containing optionally 5 % wt. % at most of cyanuric chloride,

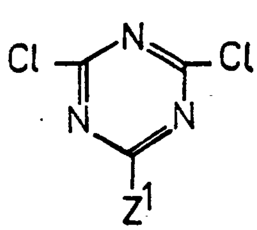

(VII),

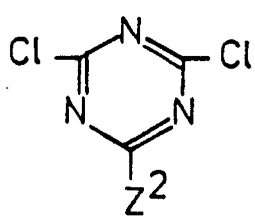

(VII A).

where $Z^1$ and $Z^2$ have the meaning as indicated in item 1, with gradually added diamines of the general formula VIII and IX

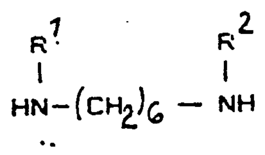

VIII)

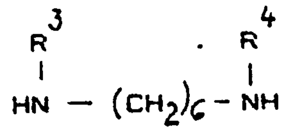

IX)

(where $R^1$,$R^2$, $R^3$ and have the above meanings) occurring within temperature interval 70 and 210° C in an inert solvent and in the presence of an inorganic base when amino compounds such as $C_1$- $C_{18}$alkyl amines, 2,2,6,6 - tetramethyl -4 - piperidine, cyclohexylamine and morpholine can be optionally added.

3. A method of synthesis according to claim 2 wherein dichlorotriazine of the general formula VII and/or VII A, containing 5 wt. % of cyanuric chloride at maximum, is polycondensed with gradually added diamines of the general formula VIII and IX, in an inert organic solvent, in the presence of an equivalent amount or of an excess (from 1 to 5 wt. % relative to the content of chlorine in dichlorotriazine derivatives and cyanuric chloride) of inorganic base, within the temperature interval from 80 to 205° C for 4 to 20 hours, the molar ratio of diamine VIII and diamine IX being from 0.95:0.05 to 0.15:0.85.

4. A method of synthesis according to claim 2 or claim 3, wehrein the total amount of dichlorotriazine of the general formula VII and/or VII A is in the excess relative to the total amount of diamines (by 1 - 25 mol. %) of the general formula VIII and IX, unreacted chlorine in triazine units of the copolymer is removed to an amount less than 0.1 wt. % by addition of amino compound (from 5 to 35 mol. % relative to the amount of dichlorotriazine VII/ or VII A), the procedure being carried out at temperatures from 140 to 210° C for 2 - 8 hours.

5. A method of synthesis according to claim 2 characterized by the polycondensation of dichlorotriazine of the general formula VII with diamine of the general formula VIII used in a molar ratio from 1:1.1 to 2, which occurs as a first step, in an inert organic solvent and in the presence of inorganic base which is used in the amount equivalent to the chlorine in dichlorotriazine and in cyanuric chloride, and in the second step, dichlorotriazine of the general formula VII and VII A is added followed by addition of diamine of the general formula IX, either at once or gradually, the total amount of diamines of the general formula VIII and IX being by 1 - 25 mol. % in the excess relative to the total amount of dichlorotriazine of the general formula VII or VII A, and after addition of inorganic base in the amount equivalent to or in the excess from 1 to 5 mol. % to dichlorotriazine added in the second step, the reaction is completed at temperatures from 150 to 210° C.

6. A method of synthesis according to claim 2 characterized by the gradual polycondensation of dichlorotriazine of the general formula VII and/or VII A, containing 5 wt. % of cyanuric chloride at maximum, with the components of the system composed of diamines of the general formula VII and IX, diamines being in a molar ratio from 0.9:0.1 to 0.15:0.85, and of amino compound in the amount from 1 to 20 mol. % relative to the total amount of the diamines used „the polycondensation being carried out in an inert organic solvent and in the presence of inorganic base which is used in the amount equivalent to the content of chlorine in dichlorotriazine and cyanuric chloride, the temperature being from 120 to 210° C, the reaction time from 4 to 15 hours.

7. A method of synthesis according to claim 2 to claim 6 wherein the total amount of diamines and amino compound of the general formula VII and IX is by 1 to 25 mol. % in excess relative to the total amount of dichlorotriazine of the general formula VII and/or VII A while inorganic base is by 1 to 5 mol. % in excess relative to the chlorine content in dichlorotriazine derivatives.

8. The use of a compound claimed in claim 1 as a stabilizer of synthetic polymers against the harmful effect of photo-oxidation degradation.

9. A polymeric blend stabilized according to claim 8 consisting of synthetic polymer and one and/or more compounds of the general formula I in the amount from 0.05 to 3 wt. % relative to the mass of synthetic polymer.

10. The polymeric blend according to claim 9 containing, in addition to the copolymer of the general formula I, other stabilizers and other common additives.

11. The polymeric blend according to claim 9 or claim 10, wherein the synthetic polymer is a polyolefin.

12. The polymeric mixture according to claim 11 wherein the synthetic polymer is polyethylene or polypropylene in the form of a foil or fibres.